# EUROPEAN PATENT APPLICATION

(11) **EP 4 811 389 A1**
(43) Date of publication of application: **23.09.2026**
(21) Application number: 26166454.4
(22) Date of filing: 20.03.2026
(51) Int. Cl.: G16H 40/67, A61B 1/00, A61B 5/00, A61C 9/00

(54) **INTRAORAL SCANNING SYSTEM AND METHOD THEREOF**

(30) Priority: 21.03.2025 DK PA202570040
(71) Applicant: 3Shape A/S, 1060 Copenhagen K (DK)
(72) Inventor: JELLINGGAARD, Anders Robert, 1060 Copenhagen K (DK)
(74) Representative: Zacco Denmark A/S

(57) **Abstract**

An intraoral scanning system (100) is disclosed that includes an intraoral scanning device (102) configured to acquire first and second light information reflected from a dental object (101). The system (100) further includes processors connected to the intraoral scanning device (102), with at least one of the processors being a remote computer (104). The processors may be configured to determine, in real-time, surface information from the first light information; generate a three-dimensional (3D) surface model (108) of the dental object (101) using the surface information; and determine internal information from the second light information. A wireless communication interface (106) communicates between the device (102) and the remote computer (104) over a wireless communication protocol including a first and a second wireless network protocol. The surface information is transmitted via the first wireless network protocol while the internal information is transmitted via the second wireless network protocol.

## Description

### FIELD OF THE INVENTION:

The present invention relates generally to intraoral scanning, and more particularly, to a system and computer-implemented method for operating an intraoral scanning system configured to acquire light information, generate real-time 3D surface models and internal information, and establish wireless communication links for data transfer.

### BACKGROUND

In the field of dentistry, obtaining accurate and detailed information about dental objects is crucial for diagnosis, treatment planning, and the fabrication of dental prosthetics. Traditional methods of capturing dental information, such as physical impressions and manual measurements, are often time-consuming and prone to errors. These methods can lead to inaccuracies in the final dental models, which may compromise the quality of dental treatments and restorations.

Existing intraoral scanning systems aim to address these challenges by providing digital solutions for capturing dental information. However, these systems face several limitations. For, many current systems struggle to process and render 3D models in real-time, leading to delays in obtaining usable data during a scanning session. This can prolong the scanning process and affect the overall efficiency of dental procedures. Further, the transmission of sensitive dental information between scanning device and processors poses significant security risks. Existing systems may not adequately protect this data, leaving it vulnerable to unauthorized access and breaches. Furthermore, wireless communication protocols used in current intraoral scanning systems may not be optimized for the real-time transmission of large amounts of data. This can result in poor quality connections and data loss, affecting the accuracy and reliability of the captured information. Moreover, some solutions exist that are limited in their ability to capture detailed internal information about dental objects. This can hinder the comprehensive analysis and diagnosis of dental conditions, as well as the precision of treatment plans. Also, maintaining a consistent quality of service during wireless data transmission is challenging. Fluctuations in wireless signal strength and interference can disrupt the scanning process and compromise the integrity of the captured data.

Therefore, there is a need for improved solutions that overcome the above challenges, and enhance the accuracy, efficiency, and security of intraoral scanning systems, thereby elevating the standard of dental care.

### SUMMARY

According to one embodiment of the present disclosure, an intraoral scanning system is disclosed. The intraoral scanning system may include an intraoral scanning device configured to acquire first light information and second light information reflected from a dental object during a scanning session. The intraoral scanning system may further include one or more processors operably connected to the intraoral scanning device, with at least one of the one or more processors being one or more remote computers. The one or more processors may be configured to: determine, in real time, surface information from the first light information; generate a three-dimensional (3D) surface model of the dental object using the surface information; and determine internal information from the second light information of the dental object. The intraoral scanning system may further include a wireless communication interface configured to communicate between the intraoral scanning device and the one or more remote computers over a wireless communication protocol. The wireless communication protocol may include a first wireless network protocol and a second wireless network protocol. The surface information may be transmitted via the first wireless network protocol and the internal information may be transmitted via the second wireless network protocol.

If the quality of the wireless connection allows for more secured wireless communication protocols between the remote external computer and the intraoral scanning device it would be beneficial to transmit the surface information and the internal information via a wireless network protocol that is a secured wireless network protocol.

In some embodiments, the first wireless network protocol may be an unsecured wireless network protocol, and the second wireless network protocol may be a secured wireless network protocol. In an embodiment, the wireless network protocol may further include a third wireless network protocol which may be a secured wireless network protocol.

In some embodiments, the one or more processors may be further configured to generate control data that includes status information of the intraoral scanning device. The control data may be transmitted via the second wireless network protocol or the third wireless network protocol.

In some embodiments, the first wireless network protocol may be configured to transmit the surface information in real-time to the one or more remote computers 104. Correspondingly, the 3D surface model of the dental object may be rendered in real-time.

In some embodiments, the one or more processors may be configured to switch between the first wireless network protocol and the second wireless network protocol or the third wireless network protocol to transmit the surface information, based on a wireless quality threshold. To this end, the one or more processors may be configured to receive Quality-of-Service (QoS) data of the wireless communication protocol during wireless transmission in the wireless communication protocol, such that the wireless quality threshold includes a level of the Quality-of-Service data. For example, the wireless quality threshold may be determined to be a level of QoS data which provides an optimal wireless connection to transmit surface information in real time via the second wireless network protocol or the third wireless network protocol.

In some embodiments, the secured wireless network protocol may be a Domain Name System (DNS) protocol or a Multicast Domain Name System (mDNS) protocol. Other examples of the secured wireless network protocol may include Internet Control Message Protocol (ICMP), Internet Group Management Protocol (IGMP), File Transfer Protocol (FTP), or Trivial File Transfer Protocol (TFTP). The unsecured wireless network protocol may be a User Datagram Protocol (UDP) or a Transmission Control Protocol (TCP); and other examples may include Secure Shell (SSH), Hypertext Transfer Protocol Secure (HTTPS), Internet Protocol Security (IPSec), or Zeroconf.

The advantage of transmitting the surface information via the unsecured wireless network protocol is that it is faster and has a lower overhead which secures a stabile real time wireless connections that allows real-time processing of the 3D surface model and real time rendering of the 3D surface model to be displayed on a displaying unit. Thus, the secured wireless network protocol has a much higher overhead dues to a very high request-data rate, so the establishing tearing down that many connections would be significant.

In some embodiment, during operation, the intraoral scanning device may broadcast, by the wireless communication interface, a unique identifier of the intraoral scanning device via the wireless communication protocol. The one or more remote computers may be configured to establish a secured wireless connection to the wireless communication interface based on the broadcasted unique identifier. Once the secured wireless connection is established between the intraoral scanning device and the one or more remote computers, an IPv4 or IPv6 address may be transmitted via a secured wireless network protocol to the one or more remote computers. Further, in some embodiments, password protected data may be transmitted from the intraoral scanning device via the secured wireless connection established between the intraoral scanning device and the one or more remote computers.

In some embodiments, the second light information may include infrared (IR) wavelengths and/or ultraviolet (UV) wavelengths. The internal information may include IR light information, UV light information and/or visible light information acquired by the intraoral scanning device. For example, the internal information may include a composition of IR light information, UV information and/or visible light information.

In some embodiments, during a first time slot of a scanning sequence of the intraoral scanning device, the second light information may include IR wavelengths, and during a second time slot of a scanning sequence of the intraoral scanning device, the second light information includes UV wavelengths.

In some embodiments, the first light information may be acquired by the intraoral scanning device at a first frame rate, and the second light information may be acquired by the intraoral scanning device at a second frame rate. The second frame rate may be lower than the first frame rate.

According to one embodiment of the present disclosure, a computer-implementable method of operating the intraoral scanning system is disclosed. The method may include acquiring first light information and second light information during a scanning session; determining, in real time, surface information from the first light information; and determining internal information from the second light information. The method may further include communicating between the intraoral scanning device and one or more processors over a wireless communication protocol. At least one of the one or more processors is one or more remote computers. The wireless communication protocol may include a first wireless network protocol and a second wireless network protocol. The method may further include transmitting, via the first wireless network protocol, the surface information; and transmitting, via the second wireless network protocol, the internal information.

Still other aspects, features, and advantages of the invention are readily apparent from the following detailed description, simply by illustrating a number of particular embodiments and implementations, including the best mode contemplated for conducting the invention. The invention is also capable of other and different embodiments, and its several details can be modified in various obvious respects, all without departing from the spirit and scope of the invention. Accordingly, the drawings and description are to be regarded as illustrative in nature, and not as restrictive.

### BRIEF DESCRIPTION OF THE DRAWINGS

The embodiments of the invention are illustrated by way of example, and not by way of limitation, in the figures of the accompanying drawings:
FIG. 1 is a block diagram of an intraoral scanning system, in accordance with some embodiments of the present disclosure,
FIG. 2 is a network communication diagram showing communication links between an operating system (OS) of an intraoral scanning device and a remote computer within the network, in accordance with some embodiments,
FIG. 3 is a flowchart of a computer-implementable method of operating the intraoral scanning system, in accordance with some embodiments of the present disclosure,
FIG. 4 illustrates an exemplary computing system (e.g. implemented as the one or more processors) that may be employed to implement processing functionality for various embodiments, and
FIG. 5 illustrates an exemplary of a system that is configured to switch between different wireless network protocols based on a wireless quality threshold.

### DESCRIPTION OF SOME EMBODIMENTS

Examples of a system, method, and computer program for generating feature data are disclosed. In the following description, for the purposes of explanation, numerous specific details are set forth in order to provide a thorough understanding of the embodiments of the invention. It is apparent, however, to one skilled in the art that the embodiments of the invention may be practiced without these specific details or with an equivalent arrangement. In other instances, well-known structures and devices are shown in block diagram form in order to avoid unnecessarily obscuring the embodiments of the invention.

The present disclosure pertains to an intraoral scanning system which is capable of capturing and processing detailed data regarding dental objects (e.g., tooth components such as enamel, dentin, pulp, and cementum, as well as supporting features including gums, periodontal ligaments, and alveolar bone, which collectively form the crown, root, and associated elements like pulp chambers and root canals). In an embodiment of the present disclosure, the intraoral scanning system may include an intraoral scanning device configured to acquire light information reflected from a dental object during a scanning session. The light information may include first light information and second light information.

The intraoral scanning device may be operably connected to one or more processors, which may include one or more remote computers. These processors may be configured to perform several key functions, including determining surface information from the first light information acquired by the scanning device, in real-time. The surface information may be used for generating a three-dimensional (3D) surface model of the dental object, that may be rendered on a display screen associated with the one or more remote computers. The real-time processing capability may enhance the efficiency of the scanning process by providing immediate feedback to a user. The one or more processors may be further configured to, as mentioned above, generate a 3D surface model of the dental object, using the surface information. The 3D surface model may be rendered in real-time, allowing for immediate visualization and analysis of the dental object.

The one or more processors may be further configured to determine internal information from the second light information. The internal information, for example, may be based on infrared (IR) light information or ultraviolet light (UV) information and/or visible light information acquired by the intraoral scanning device.

It may be noted that the surface information may relate to the external characteristics of dental objects, such as tooth shape, enamel texture, and gum line contours, that can be captured using visible light. In contrast, internal information includes the subsurface layers of dental objects (e.g. such as dentin, pulp, or root canal details) that are not visible to the naked eye and lie below the surface. As such, the internal information may be captured using infrared or UV light. Together, these two types of data provide a comprehensive view of dental health and structure, with surface information revealing external form and internal information uncovering the concealed conditions beneath.

The intraoral scanning system may further include a wireless communication interface configured to facilitate communication between the intraoral scanning device and the one or more remote computers. For example, the one or more remote computers may include a rendering device configured to render a 3D surface model of the dental object in real-time. This rendering device may, therefore, by communicatively coupled with the intraoral scanning device, via the wireless communication interface.

The communication between the intraoral scanning device and the one or more remote computers (e.g. the rendering device) may occur over a wireless communication protocol. In some embodiments, the wireless communication protocol may include a first wireless network protocol and a second wireless network protocol. Further, in some embodiments, the surface information may be transmitted via the first wireless network protocol, while the internal information may be transmitted via the second wireless network protocol.

The first wireless network protocol may be adapted for real-time data transmission, ensuring that the surface information is processed and rendered without delay. Further, in some embodiments, the first wireless network protocol may be unsecured to optimize speed and efficiency. For example, the first wireless network protocol may be a User Datagram Protocol (UDP). UDP is a lightweight, connectionless transport layer protocol in the Internet Protocol suite, capable of fast, efficient data transmission where reliability is less critical than speed. The second wireless network protocol may place greater emphasis on security, ensuring that sensitive internal information is protected during transmission. As such, the second wireless network protocol may be secured to prevent unauthorized access and data breaches.

The intraoral scanning system may be configured to generate control data, which may include status information of the intraoral scanning device. For example, the status information. By way of example, the status information may include power status (e.g. whether the device is powered ON, battery level, or whether the device is charging), connection status (e.g. whether the device is connected to wireless network), calibration status, scanning status (e.g. showing "Ready to scan", "Scanning in progress", "Scan completed", etc.), errors or alerts, hardware status (e.g. device temperature), software integration status (e.g. related to software updates), etc. The control data may be transmitted via the second wireless network protocol or a third wireless network protocol, ensuring secure and reliable communication. The third wireless network protocol may also be a secured wireless network protocol. By way of an example, the second and the third wireless network protocol may be a Domain Name System (DNS)-based or Multicast DNS (mDNS)-based protocol, enabling unsecured data transmission. DNS may be a hierarchical, distributed system that relies on centralized servers to resolve domain names (e.g., www.xyz.com) into IP addresses, functioning globally across the Internet with queries directed to authoritative name servers. Further, mDNS may be a decentralized protocol designed for local networks, enabling devices to resolve hostnames (e.g., abc.local) without a central server by multicasting queries to all devices on the same subnet, which respond if they match the requested name.

The one or more processors may be capable of switching between wireless network protocols based on a wireless quality threshold, thereby allowing for optimal data transmission even in varying wireless conditions. In some embodiments, the intraoral scanning system may be configured to allow transmission of password protected data from the intraoral scanning device, via the secured wireless connection established between the intraoral scanning device and the one or more remote computers, thereby enhancing the security of sensitive information.

In some embodiments, the one or more processors may be configured to dynamically switch, between a first wireless network protocol, a second wireless network protocol, and optionally a third wireless network protocol, to transmit surface information obtained from an intraoral scanning device. For example, the switching may be based on a predetermined wireless quality threshold. The wireless quality threshold may be defined by one or more performance metrics, including but not limited to signal strength, latency, packet loss rate, or available bandwidth, which the processor continuously or periodically monitors to assess the suitability of the active wireless protocol. The processors may be adapted to seamlessly transition between the first, second, and third wireless network protocols in response to real-time network conditions, thereby ensuring uninterrupted and efficient transmission of the surface information to a receiving device, such as a remote computer.

It should be noted that the one or more processors units configured to perform the switching function may be implemented in various configurations depending on the system architecture. For example, these processors may be implemented in the intraoral scanning device, enabling onboard decision-making and protocol switching for a compact, self-contained solution; or, within the wireless communication interface; or, within the one or more remote computers, such as a cloud server or local workstation, centralizing control. As such, the implementation of the processors may be based on the specific use cases, balancing factors such as device size, power efficiency, and network complexity, while maintaining robust and adaptive wireless communication of the surface information.

In some embodiments, the intraoral scanning device may broadcast a unique identifier (e.g., a Media Access Control (MAC) address or Universally Unique Identifier (UUID)) via the wireless communication interface, enabling the remote computer(s) to establish a secured wireless connection. Upon receiving the unique identifier, the associated remote computer(s) may initiate a handshake protocol (e.g., via mDNS or DNS), authenticating the device and establishing a communication link. Further, once the communication link is established, an IPv4 or IPv6 address may be assigned and transmitted to the remote computer(s) over via a secured wireless network protocol, thereby facilitating subsequent data exchange. In some embodiments, the IPv4 or IPv6 address may be established using Dynamic Host Configuration Protocol (DHCP).

In some embodiments, password-protected data may be transmitted exclusively over the secured wireless network protocol, thereby ensuring compliance with privacy standards (e.g., HIPAA). For example, the password-protected data may include as patient-specific internal information or device configuration settings,

During operation, the intraoral scanning device may be configured to acquire the second light information reflected from the dental object using a time-division approach within a scanning sequence. For instance, during a first time slot of the scanning sequence, the second light information may include IR wavelengths, and during a second time slot of the same scanning sequence, the second light information may include UV wavelengths. This segmentation of the IR-based and UV-based data acquisition may enhance the ability of the system to collect multi-spectral internal information about the dental object, such as subsurface structural details or material properties, while maintaining compatibility with the high-speed acquisition of first light information (e.g., visible light).

The present disclosure further describes a computer-implementable method of operating an intraoral scanning system. The method may include acquiring the first light information and the second light information during a scanning session, and determining surface information from the first light information and internal information from the second light information in real time. The obtained data may be communicated between the intraoral scanning device and one or more processors, including remote computers, over a wireless communication protocol. The surface information may be transmitted over the first wireless network protocol and the internal information may be transmitted over the second wireless network protocol.

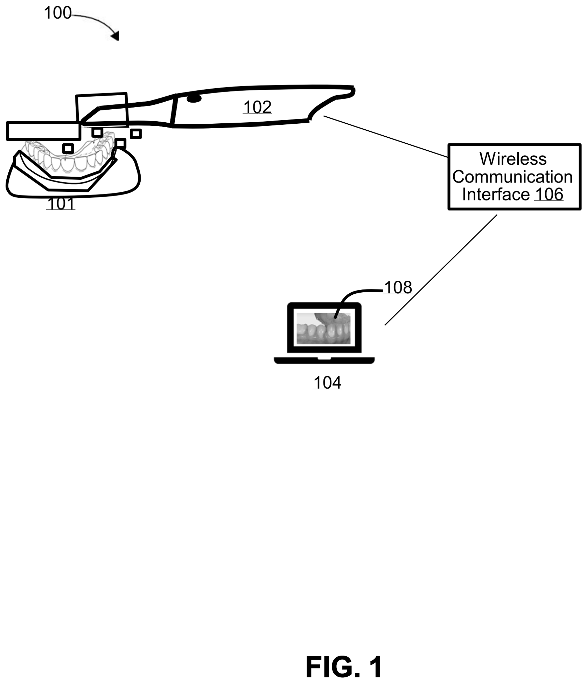

Referring now to **FIG. 1****,** a block diagram of an intraoral scanning system 100 is illustrated, in accordance with some embodiments of the present disclosure. The intraoral scanning system 100 (hereinafter, also referred to as "system 100") may be configured to acquire and process light information from a dental object 101 to generate three-dimensional 3D surface models and internal information, utilizing a multi-protocol wireless communication framework.

As shown in FIG. 1, the system 100 may include an intraoral scanning device 102 (hereinafter, also referred to as "device 102") which may be a handheld or portable apparatus positioned proximate to the dental object 101. The dental object 101, as illustrated in FIG. 1, may be a tooth or a portion of oral tissue (e.g., gums or a dental arch). During a scanning session, the device 102 may transmit light on the dental object 101, and the light, upon reflection from the dental object 101, may be captured by the device 102. To this end, the device 102 may include with one or more light sources and sensors (not shown in FIG. 1) configured to acquire first light information and second light information reflected from the dental object 101. In an embodiment, the first light information may be based on visible light (e.g., wavelengths of 400-700 nm), and may be emitted by a light source such as a white LED. The visible light may be captured by a sensor (e.g., a CMOS or CCD sensor). The second light information may be based on infrared (IR) wavelengths (e.g., 850 nm) and/or ultraviolet (UV) wavelengths (e.g., 365 nm), and may be generated by respective IR and UV light sources (e.g., LEDs or laser diodes), and detected by corresponding sensors (e.g., IR-sensitive photodiodes or UV-filtered detectors). As such, the internal information derived from this second light information may include IR light information (e.g., thermal signatures), UV light information (e.g., fluorescence data), and/or visible light information (e.g., from residual first light processing), collected by the device 102 to provide a multi-faceted view of the dental object's internal characteristics. The internal information may include a composition of IR, UV, and/or visible light information, that may be processed by the one or more processors using techniques like spectral fusion or weighted averaging to integrate these datasets into a comprehensive profile. Therefore, by combining thermal, fluorescence, and structural insights, diagnostic accuracy can be enhanced.

The system 100 may further include one or more processors operably connected to the device 102. At least one of these processors may be implemented as a remote computer 104, in accordance with one embodiment, as shown in FIG. 1. The remote computer 104, for example, may be implemented as a workstation, a laptop, or a cloud server, positioned at a distance from the device 102. The one or more processors may be configured to perform one or more real-time functions. For example, the one or more processors may determine surface information from the first light information by processing the first light information. To this end, the first light information may be processed using techniques such as structured light triangulation or stereophotogrammetry, extracting spatial coordinates and surface texture of the dental object 101. The surface information may be later used to generate a three-dimensional 3D surface model 108 of the dental object 101, which may be which may be rendered and displayed on a screen associated with the remote computer 104. Further, the one or more processors may determine internal information from the second light information, based on analyzing IR and/or UV reflections. The internal information may relate to subsurface features (e.g., enamel thickness, caries) or material properties.

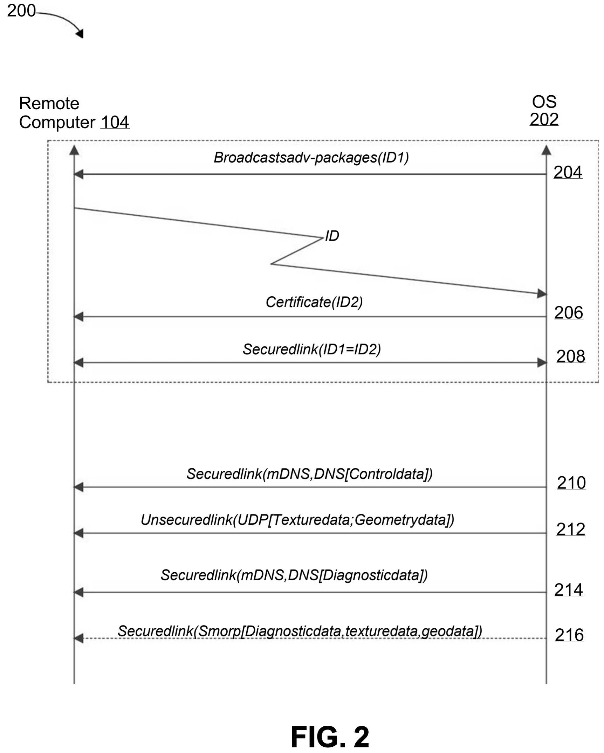

As shown in FIG. 1, the system 100 may further include a wireless communication interface 106 which may be configured to facilitate communication between the device 102 and the remote computer(s) 104. To tr 106 may implement a wireless communication protocol for facilitating communication between the device 102 and the remote computer(s) 104. The wireless communication protocol may include a first wireless network protocol, a second wireless network protocol, and optionally, a third wireless network protocol. The wireless network protocols are further explained in detail in conjunction with FIG. 2.

In some embodiments, the surface information may be transmitted via the first wireless network protocol, while the internal information may be transmitted via the second wireless network protocol. The first wireless network protocol may be adapted for real-time data transmission, ensuring that the surface information is processed and rendered without delay. The first wireless network protocol may be a high-bandwidth and low-latency pathway, adapted to deliver voluminous and real-time surface data required for the 3D surface model. Further, in some embodiments, the first wireless network protocol may be unsecured to optimize speed and efficiency. For example, the first wireless network protocol may be based on a User Datagram Protocol (UDP) or a Transmission Control Protocol (TCP). UDP is a lightweight, connectionless Internet protocol that prioritizes speed and low latency, transmitting data packets (datagrams) without establishing a formal connection or guaranteeing delivery, making it ideal for real-time applications like streaming surface information (e.g., at 2500 FPS) via the first wireless network protocol. TCP is a connection-oriented protocol that ensures reliable, ordered delivery of data by establishing a handshake, retransmitting lost packets, and managing flow control, which suits scenarios requiring data integrity, such as transmitting internal information or control data via the second wireless network protocol, though at the cost of higher latency (e.g., 20-50 ms). It should be noted that other than UDP and TCP, the first wireless network protocol can also be selected from Internet Control Message Protocol (ICMP), Internet Group Management Protocol (IGMP), File Transfer Protocol (FTP), or Trivial File Transfer Protocol (TFTP).

The second wireless network protocol, on the other hand, may place greater emphasis on security, ensuring that sensitive internal information is protected during transmission. As such, the second wireless network protocol may be secured to prevent unauthorized access and data breaches. The second wireless network protocol may include a secured Domain Name System (DNS) or Multicast DNS (mDNS) protocol. DNS protocol is traditionally used to resolve domain names to IP addresses, and can be secured with extensions like DNS over TLS (DoT) or DNS over HTTPS (DoH), employing encryption and authentication to safeguard data exchanges. mDNS - a variant of DNS - enables device discovery and communication without a central server (e.g., using the .local domain), and can be paired with security mechanisms like TLS or DNSCrypt to provide encrypted, multicast-based data transmission ideal for secure, short-range connections. Other than DNS and mDNS, the second wireless network protocol may be implemented through secured network protocols like Secure Shell (SSH), Hypertext Transfer Protocol Secure (HTTPS), Internet Protocol Security (IPSec), or Zeroconf.

During operation, the device 102 may capture multi-spectral light information from the dental object 101. In some embodiments, the first light information may be acquired at a first frame rate, while the second light information may be acquired at a second frame rate, i.e. the first and the second light information may be acquired at different rates. In particular, the device 102 may obtain the first light information at a high frame rate, for example, 2500 frames per second (FPS). The high FPS associated with the first light information may be required for capturing the surface information related to the dental object 101. The device 102 may capture visible light at 2500 FPS to provide high-speed and detailed imaging. In some embodiments, the visible light may be captured as 100-frame bursts occurring 25 times per second, to thereby allow for obtaining multiple (i.e. 100) images at one point in time, out of which, the images with the best pixel quality may be selected for analysis and rendering. The second frame rate, for example, may be 500 FPS; in other words, the second light information (UV or IR) light may be captured at one-fifth the rate of the first light information.

The scanning sequence refers to a repetitive cycle of data acquisition performed by the intraoral scanning device 102 during a scanning session, where each cycle is divided into discrete time slots to manage the collection of different types of light information. In one exemplary embodiment, the scanning sequence operates at a frequency aligned with the second frame rate (e.g., 500 frames per second, as described in prior embodiments), resulting in a cycle duration of approximately 2 milliseconds (ms) per frame. Within each cycle, the sequence is segmented into at least two time slots: a first time slot dedicated to IR wavelength acquisition and a second time slot dedicated to UV wavelength acquisition. For instance, the first time slot may span the initial 1 ms of the cycle (e.g., 0-1 ms), during which the IR light source is activated to emit IR light toward the dental object (101), and the reflected IR wavelengths are captured by the IR sensor. Subsequently, the second time slot may span the remaining 1 ms of the cycle (e.g., 1-2 ms), during which the UV light source is activated, and the reflected UV wavelengths are captured by the UV sensor. The timing control unit ensures precise synchronization, deactivating one light source before activating the next to prevent spectral overlap and ensure clean separation of IR and UV data.

In some embodiments, during a scanning operation, the device 102 may capture IR wavelengths in a first time slot and UV wavelengths in a second time slot corresponding to the second light information. In other words, the IR and UV wavelengths may not be captured at the same point in time, and instead may be captured in a sequential manner in different time slots. As it will be appreciated by those skilled in the art, capturing IR (e.g., 850 nm) and UV (e.g., 365 nm) wavelengths at the same time may lead to interference, as deeper penetration of the IR wavelengths may scatter into the UV sensor's range, and UV-induced fluorescence may overlap with IR detection. As a result, the signal-to-noise ratio may degrade that may compromise the accuracy of internal information. As such, capturing the IR and UV wavelengths at distinct time slots may prevent cross-talk and preserve data fidelity, thereby ensuring high-quality internal diagnostics alongside real-time surface modeling in the system 100.

Further, acquisition of the IR wavelengths during the first time slot enables the system 100 to gather internal information related to structural features of the dental object 101. For example, IR light, due to its longer wavelength and greater penetration depth (as compared to visible light) may allow for detecting subsurface anomalies, such as enamel thinning, dentin exposure, or thermal gradients indicative of inflammation or decay. Similarly, the acquisition of UV wavelengths during the second time slot may allow for leveraging ability of the UV light to induce fluorescence or reveal material-specific properties. For instance, UV illumination may cause dental tissues or bacterial deposits to fluoresce, with the emitted wavelengths being captured by the UV sensor to identify caries, plaque, or composite restorations. Therefore, the one or more processors may analyze these UV reflections, in conjunction with IR data, to construct a composite internal profile of the dental object.

The first light information, in contrast, may be collected concurrently throughout the scanning session. In one embodiment, the first light information (i.e. via the visible light) may be obtained in parallel with the IR and UV acquisitions. In alternative embodiments, the scanning sequence may include additional time slots for visible light, such as a third time slot per cycle. A timing control may be implemented to ensure that the IR and UV acquisitions do not interfere with the visible light acquisitions.

Once the first and the second light information is obtained by the device 102, the same may be transmitted to the remote computer 104 based on establishing a communication link between the device 102 and the remote computer 104. To this end, the device 102 may broadcast a unique identifier (e.g., a distinct code or address assigned to the intraoral scanning device) of the device 102 via the wireless communication protocol implemented via the wireless communication interface 106. The broadcasting may allow the one or more remote computers 104 to establish a secured wireless connection based on the unique identifier. Additionally, the process of establishing the communication link may include authentication steps or encryption protocols to ensure data integrity and privacy. The use of the unique identifier may simplify the pairing process and help prevent unauthorized access by ensuring that only recognized device 102 can connect, thereby enhancing the overall security and efficiency of the communication between the device 102 and the remote computer(s) 104.

The IR and UV data collected during their respective time slots may be processed by the one or more processors. The one or more processors may be implemented in the intraoral scanning device, or within the wireless communication interface, or within the one or more remote computers. For example, the one or more processors may apply spectral analysis techniques (such as Fourier transforms for IR data) to isolate thermal signatures or fluorescence intensity mapping for UV data, to thereby derive the internal information. The internal information may be then transmitted via the second wireless network protocol to the remote computer(s) 104. The second wireless network protocol may be a secured protocol (e.g., mDNS). The time-slotted approach reduces the data rate demands on the second protocol compared to the first protocol (e.g., UDP for surface information), aligning with the lower second frame rate (e.g., 500 FPS) and ensuring efficient bandwidth utilization. The wireless network protocol may further include a third wireless network protocol, which again may be a secured wireless network protocol. In some embodiments, the one or more processors may generate control data that includes status information of the device 102. The control data may be transmitted via the second wireless network protocol or the third wireless network protocol. The first wireless communication protocol includes a first security level and the second wireless communication protocol includes a second security level. The first security level is different from the second security level. The first and second security level provide a level of security in the communication that affects respectively the latency in the first and second wireless communication protocol differently. The second security level may provide less latency in the communication than the first security level. The latency in communication is detrimental for the real time processing and displaying of the three-dimensional model on a displaying unit. Different types of data and/or scan data is transmitted between the intraoral scanning device and the client device and some of the scan data is relevant for the three-dimensional surface model which real time processing and displaying is detrimental for a user experience point of view, and the protocol being used for transmitting scan data for the three-dimensional model may have a lower level of security than other type of data and/or scan data (i.e. surface information) for the purpose of achieving the real time displaying/updating of the three-dimensional model on the displaying unit. The first and second security levels may correspond to a secured and unsecured wireless network protocol, respectively. The quality of the connection (i.e. connection quality) between the intraoral scanning device and the client device may change during use of the intraoral scanning device or due to change in location of the intraoral scanning device or the client device. Due to the change in connection quality the level of security in the first security level and/or the second security level may change to different levels by adding or removing steps or complexity of the secured/unsecured wireless network protocol, i.e. security layer optimization. For example, the surface information may comprise a header, a timestamp and a scan data layer. Ideally, all three layers should be encrypted but if the connection quality drops to a level where the real time communication between the intraoral scanning device and the client device starts to be affected negatively, then one or more of the three layers should be unencrypted. The scan data layer should always be encrypted. In another example the variation in the security level may be provided by changing the security layer. For example, the security layer in the secured/unsecured wireless network protocol may change between TLS handshake, session resumption, packet encryption or authentication. For example, typical latency provided by TLS handshake may be between 50 to 150 ms. Session resumption may introduce latency of less than 10 ms, packet encryption or authentication may introduce latency of less than 1 ms. Which means, if the connection quality drops the secured/unsecured wireless network protocol the one or more processors may apply changes to the security layer for example by disabling TLS handshake and instead enabling authentication.

In some embodiments, the one or more processors may be configured to receive Quality-of-Service (QoS) data of the wireless communication protocol during wireless transmission in the wireless communication protocol. Further, a wireless quality threshold may be determined to include a level of the QoS data. The QoS data may include a set of measurable parameters that characterize the performance and reliability of the network connection, and may be based on signal strength (e.g., in decibel-milliwatts, dBm), latency (e.g., in milliseconds), packet loss rate (e.g., as a percentage), and bandwidth availability (e.g., in megabits per second, Mbps). These parameters may be monitored in real-time by the one or more processors via the wireless communication interface 106 to assess the effectiveness of the wireless protocols. The wireless quality threshold may be determined to be a level of the QoS data which provides an optimal wireless connection to transmit surface information in real time via the second wireless network protocol or the third wireless network protocol. For example, a signal strength of -100 dBm, latency below 25 ms, or packet loss under 5% may define a threshold for optimal transmission of surface information.

Therefore, the one or more processors may rely on the QoS data for switching between the second and third wireless protocols, to thereby maintain a smooth user experience with respect to data transmission. For example, the one or more processors may switch, based on a wireless quality threshold, between the first wireless network protocol, and the second wireless network protocol or the third wireless network protocol to transmit the surface information. In other words, when the QoS data is indicative of signal wireless quality above the threshold, the one or more processors may even cause to transmit the surface data via a secured communication link, i.e. the second or third wireless network protocol.

In some embodiments, the device 102 may implement an Operating System (OS) which may initiate network communication by broadcasting a unique identifier. In other words, the device 102 may broadcast a unique identifier of the device 102 through the wireless communication interface 106, via the wireless communication protocol. In response to the broadcasting, the one or more remote computers 104 may to establish a secured wireless connection to the wireless communication interface 106 based on the broadcasted unique identifier. In particular, for example, the OS may broadcast advertisement packages comprising the unique identifier via the wireless communication interface 106. The unique identifier, for example, may include a Media Access Control (MAC) address or Universally Unique Identifier (UUID), associated with the device 102.

Following the receipt of the advertisement package, wireless connection may be established between the remote computer(s) 104 and the (IOS of) the device 102 through wireless network protocol. This connection may be based on a handshake process to initiate direct communication. The IOS may transmit a certificate to the remote computer(s) 104 via the wireless network protocol; the certificate may include authentication data, such as a digital signature or serial number, issued by a trusted authority or embedded in the device 102 during manufacturing. The remote computer(s) 104 may receive the certificate and validate it by comparing the serial number to that received in the advertisement package, ensuring the broadcasting device matches the connecting device to prevent unauthorized access. If the serial numbers match, a secured communication link is established, leveraging encryption protocols like Transport Layer Security (TLS) or Secure Sockets Layer (SSL) to protect against interception or tampering. In some embodiments, ICMP may be used for ping communication. In other words, the IOS 202 may use ICMP to interact with the remote computer 104 to inform that the IOS 202 intends to be connected to the remote computer 104.

Once the secured connection is established between the device 102 and the remote computer(s) 104), an IPv4 or IPv6 address may be transmitted from the device 102 to the remote computer(s) 104 via a secured wireless network protocol, such as the second wireless network protocol. For example, this IP address may be assigned dynamically (e.g., via DHCP) or statically embedded in the firmware of the device 102, serves as a unique network identifier. The transmission may occur post-authentication to ensure security, leveraging the encryption (e.g., AES-256) to protect the address from interception, thereby establishing a stable, addressable connection.

Further, in some embodiments, password protected data may be transmitted from the device 102 via the secured wireless connection established between the device 102 and the remote computer(s) 104. The password protected data, which may include sensitive internal information, patient-specific records, or device configuration settings, may be encrypted and locked with a password or cryptographic key stored within the firmware of the device 102, ensuring that only authorized the remote computer(s) 104 with the matching credentials can decrypt and access it. This feature, therefore, enhances data security, protecting against unauthorized interception or access during transmission, thereby complying with privacy standards (e.g., HIPAA).

Referring now to **FIG.** 2, a network communication diagram 200 is illustrated showing communication links between an operating system (OS) 202 of the intraoral scanning device 102 and the remote computer 104 within the network, in accordance with some embodiments. FIG. 2 shows the process of establishing a secure wireless connection for transmitting data, such as surface information or internal information, from the IOS 202 to the remote computer 104. Further, FIG. 2 represents the sequence of advertisement broadcasting, connection establishment, certificate verification, and secured/unsecured data transmission, showing the distinct communication links and protocols involved.

The network communication between the IOS 202 (or the device 102) and the remote computer 104 may be enabled by the wireless communication interface 106 which may be capable of broadcasting, establishing a connection via link, and transmitting secured data. The IOS 202 may be a custom firmware or embedded operating system implemented on the device 102. The remote computer 104 may be equipped with compatible network hardware and software to receive, validate, and process the data, supported by a processor and memory for real-time operations.

As shown in FIG. 2, the IOS 202 of the device 102 may initiate network communication by broadcasting advertisement packages, via a communication link 204. The advertisement package may include a unique identifier (e.g., a Media Access Control (MAC) address or Universally Unique Identifier (UUID)) associated with the device 102. This broadcast is represented by a unidirectional arrow or wave pattern from the IOS 202 to the remote computers, indicating that it is an open signal receivable by any remote computer in range. As a result, one of the remote computers 104 is able to identify the device 102 within the network.

Following the receipt of the advertisement package, a wireless connection may be established between the remote computer 104 and the IOS 202 via a communication link 206, based on a handshake process to initiate direct communication. The IOS 202 may transmit a certificate to the remote computer 104 via the communication link 206. For example, the certificate may include authentication data, such as a digital signature or serial number, issued by a trusted authority or embedded within the IOS during manufacturing. The remote computer 104 may receive this certificate and perform a validation check by comparing the serial number within the certificate to the serial number previously received in the advertisement package via the link 204, ensuring that the device broadcasting the advertisement matches the one requesting a connection, thereby preventing unauthorized access. If the serial number in the certificate matches the serial number stored in the advertisement package, the remote computer 104 establishes a secured communication link with the IOS 202. This secured link may use encryption protocols (e.g., Transport Layer Security (TLS) or Secure Sockets Layer (SSL)), to enable protection against interception or tampering. If the serial numbers do not match, the connection attempt may be terminated.

Once the secured connection is established, transmission of the remaining data from the advertisement package (i.e. plexus package) may occur from the IOS 202 to the remote computer 104 via a secured communication link 208. The communication link 208 may be based on a secured mDNS or DNS protocol. The plexus package may include additional configuration data, status information (e.g., device battery level, sensor calibration), or a portion of the surface or internal information. The data transmission through the communication link 208 may be encrypted and transmitted only after authentication, for ensuring that sensitive or operational data is exchanged only over a verified and protected connection.

As mentioned above, the wireless communication interface 106 may communicate between the IOS 202 (i.e. the device 102) and the remote computer 104 over wireless communication protocol. The wireless communication protocol may include a first wireless network protocol, a second wireless network protocol, and a third wireless network protocol.

### First Wireless Network Protocol

The first wireless network protocol may be an unsecured wireless network protocol, and may be used for transmitting the surface information (e.g., texture and geometry data associated with the dental object 101) from the IOS 202 to the remote computer 104. In particular, the first wireless network protocol may be configured to transmit the surface information in real-time to the remote computer 104, so as to enable rendering of the 3D surface model 108 of the dental object 101 in real-time, on the remote computer 104. As shown in FIG. 2, the first wireless network protocol may be implemented through a communication link 212. For example, the communication link 212 may be based on a User Datagram Protocol (UDP) - a lightweight, connectionless transport layer protocol, capable of fast data transmission. The communication link 212 may be a high-bandwidth and low-latency pathway, adapted to deliver voluminous and real-time surface data required for the 3D surface model.

### Second Wireless Network Protocol

The second wireless network protocol may be a secured wireless network protocol. In an implementation, the second wireless network protocol may be used for transmitting the internal information (e.g. diagnostic data) associated with the dental object 101. For example, as shown in FIG. 2, the second wireless network protocol may be implemented through a communication link 214. For example, the communication link 214 may be based on a secured Domain Name System (DNS) or Multicast DNS (mDNS) protocol adapted to place greater emphasis on security, ensuring protection of sensitive information during transmission and prevention of unauthorized access and data breaches.

In some embodiments, the control data including status information of the device 102 may be transmitted via the second wireless network protocol. To this end, the second wireless network protocol may also be implemented through a communication link 210, which, for example, may be based on a secured Domain Name System (DNS) or Multicast DNS (mDNS) protocol. The communication link 210, therefore, may be used to transmit the control data that includes status information of the device 102.

In some embodiments, the one or more processors may be configured to receive Quality-of-Service data of the wireless communication protocol during wireless transmission in the wireless communication protocol. A wireless quality threshold may be determined so as to include a level of the Quality-of-Service data. The QoS data may include a set of measurable parameters that characterize the performance and reliability of the network connection, and may be based on parameters including signal strength (e.g., in decibel-milliwatts, dBm), latency (e.g., in milliseconds), packet loss rate (e.g., as a percentage), and bandwidth availability (e.g., in megabits per second, Mbps). These parameters may be monitored in real-time by the one or more processors via the wireless communication interface 106 to assess the effectiveness of the wireless protocols.

The wireless quality threshold may be determined to be a level of Quality-of-Service data which provides an optimal wireless connection to transmit surface information in real time via the second wireless network protocol or the third wireless network protocol. For example, a signal strength of -100 dBm, latency below 25 ms, or packet loss under 5% may define a threshold for optimal transmission of surface information. Therefore, QoS data may be relied on for switching between the second and third wireless protocols, to thereby maintain a smooth user experience with respect to data transmission. As such, the one or more processors may switch, based on a wireless quality threshold, between the first wireless network protocol, and the second wireless network protocol or the third wireless network protocol to transmit the surface information. In other words, when the QoS data is indicative of signal wireless quality above the threshold, the one or more processors may even cause to transmit the surface data via a secured communication link. In such scenarios of high signal wireless quality, the second wireless network protocol may also be implemented through a communication link 216 (communication link 216 is shown in dotted line, as this link may be active only in scenarios of high signal wireless quality). The communication link 216 may be used for transmitting the surface information (i.e. geometric and texture data) or the internal information associated with the dental object 101. For example, the communication link 216 may be based on a proprietary wireless network protocol (also referred to as "Smorp"), capable of providing secured data transmission.

### Third Wireless Network Protocol

The third wireless network protocol may be a secured wireless network protocol. In some embodiments, the control data including status information of the device 102 may be transmitted via the third wireless network protocol. To this end, the third wireless network protocol may be implemented through the communication link 210, which, for example, may be based on a secured DNS or mDNS protocol. The communication link 210, therefore, may be used to transmit the control data that includes status information of the device 102.

Further, as mentioned above, the one or more processors may rely on QoS data for switching between the second and third wireless protocols, to thereby maintain a smooth user experience with respect to data transmission. As such, when the QoS data is indicative of signal wireless quality above the threshold, the one or more processors may cause to transmit the surface data via a secured communication link, for example, the communication link 210 implementing the third wireless network protocol.

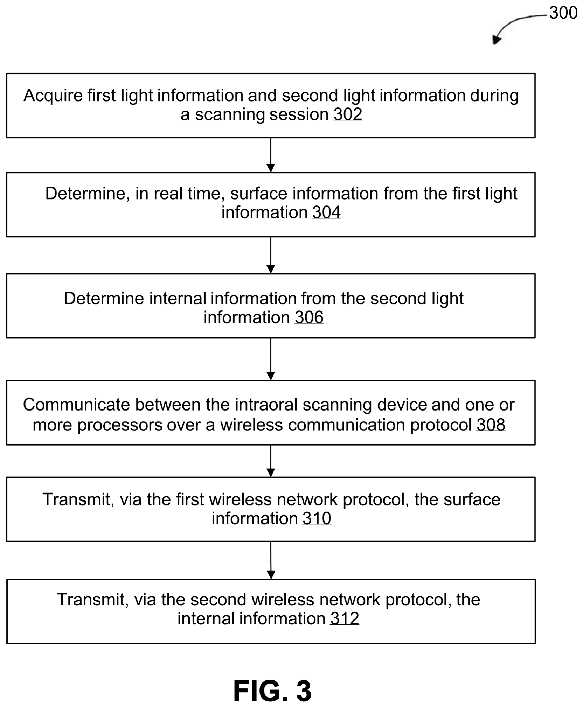

Referring now to **FIG. 3****,** a flowchart of a computer-implementable method 300 is illustrated, in accordance with some embodiments of the present disclosure. The method 300, for example, may be performed by the one or more processors including at least one remote computer 104, as already explained above.

At step 302, the intraoral scanning device 102 may acquire the first light information and second light information reflected from the dental object 101 during a scanning session. The first light information, for example, visible light (e.g., 400-700 nm), may be captured at a high frame rate (e.g., 2500 FPS) using a light source (e.g., white LED) and sensor (e.g., CMOS). The second light information, comprising IR (e.g., 850 nm) and/or UV wavelengths (e.g., 365 nm), may be acquired at a lower frame rate (e.g., 500 FPS) with dedicated IR and UV sources and sensors.

At step 304, the one or more processors may determine surface information from the first light information in real-time. To this end, the first light information may be analysed using techniques such as structured light triangulation or photogrammetry to extract spatial coordinates and texture details of the surface of the dental object 101 (e.g., teeth or gums).

At step 306, the one or more processors may determine internal information from the second light information. To this end, the IR and/or UV data may be processed to reveal subsurface features (e.g., enamel thickness from IR) or material properties (e.g., fluorescence indicating caries from UV), for example, using spectral analysis or multi-wavelength fusion techniques.

At step 308, communication between the device 102 and the at least one remote computer 104 may be established, over a wireless communication protocol. The wireless communication protocol may include the first wireless network protocol (e.g., UDP or TCP), and the second wireless network protocol (e.g., DNS or mDNS).

At step 310, the surface information may be transmitted via the first wireless network protocol to the remote computer(s) 104. The first wireless network protocol - a high-bandwidth and low-latency protocol - ensures real-time delivery of the voluminous surface data for immediate 3D surface modelling and rendering.

At step 312, the internal information may be transmitted via the second wireless network protocol to the remote computer(s) 104. The second wireless network protocol - a secured protocol - protects the potentially sensitive diagnostic data.

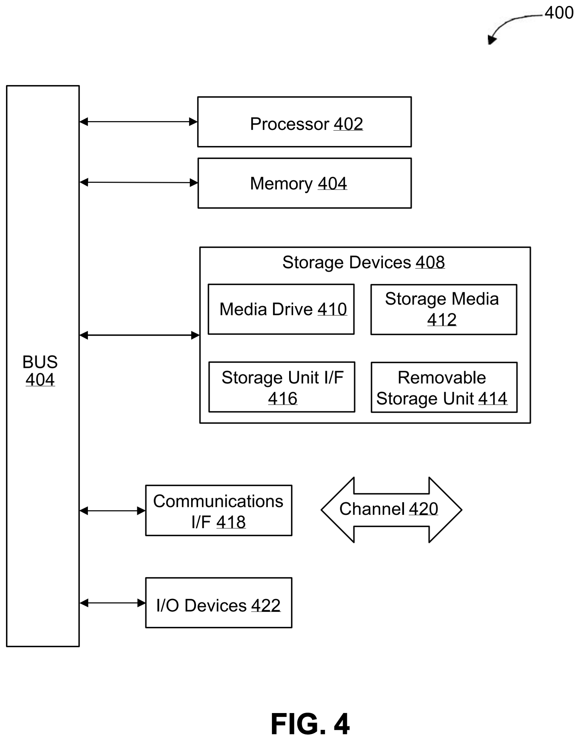

Referring now to **FIG. 4****,** an exemplary computing system 400 (e.g. implemented as the one or more processors) that may be employed to implement processing functionality for various embodiments (e.g., as a SIMD device, client device, server device, one or more processors, or the like) is illustrated. Those skilled in the relevant art will also recognize how to implement the invention using other computer systems or architectures. The computing system 400 may represent, for example, a user device such as a desktop, a laptop, a mobile phone, personal entertainment device, DVR, and so on, or any other type of special or general-purpose computing device as may be desirable or appropriate for a given application or environment. The computing system 400 may include one or more processors, such as a processor 402 that may be implemented using a general or special purpose processing engine such as, for example, a microprocessor, microcontroller or other control logic. In this example, the processor 402 is connected to a bus 404 or other communication media. In some embodiments, the processor 402 may be an Artificial Intelligence (AI) processor, which may be implemented as a Tensor Processing Unit (TPU), or a graphical processor unit, or a custom programmable solution Field-Programmable Gate Array (FPGA).

The computing system 400 may also include a memory 406 (main memory), for example, Random Access Memory (RAM) or other dynamic memory, for storing information and instructions to be executed by the processor 402. The memory 406 also may be used for storing temporary variables or other intermediate information during the execution of instructions to be executed by processor 402. The computing system 400 may likewise include a read-only memory ("ROM") or other static storage device coupled to bus 404 for storing static information and instructions for the processor 402.

The computing system 400 may also include storage devices 408, which may include, for example, a media drive 410 and a removable storage interface. The media drive 410 may include a drive or other mechanism to support fixed or removable storage media, such as a hard disk drive, a floppy disk drive, a magnetic tape drive, an SD card port, a USB port, a micro-USB, an optical disk drive, a CD or DVD drive (R or RW), or other removable or fixed media drive. A storage media 412 may include, for example, a hard disk, magnetic tape, flash drive, or other fixed or removable media that is read by and written to by the media drive 410. As these examples illustrate, the storage media 412 may include a computer-readable storage medium having stored therein particular computer software or data.

In alternative embodiments, the storage devices 408 may include other similar instrumentalities for allowing computer programs or other instructions or data to be loaded into the computing system 400. Such instrumentalities may include, for example, a removable storage unit 414 and a storage unit interface 416, such as a program cartridge and cartridge interface, a removable memory (for example, a flash memory or other removable memory module) and memory slot, and other removable storage units and interfaces that allow software and data to be transferred from the removable storage unit 414 to the computing system 400.

The computing system 400 may also include a communications interface 418. The communications interface 418 may be used to allow software and data to be transferred between the computing system 400 and external devices. Examples of the communications interface 418 may include a network interface (such as an Ethernet or other NIC card), a communications port (such as for example, a USB port, a micro-USB port), Near field Communication (NFC), etc. Software and data transferred via the communications interface 418 are in the form of signals which may be electronic, electromagnetic, optical, or other signals capable of being received by the communications interface 418. These signals are provided to the communications interface 418 via a channel 420. The channel 420 may carry signals and may be implemented using a wireless medium, wire or cable, fiber optics, or other communications medium. Some examples of the channel 420 may include a phone line, a cellular phone link, an RF link, a Bluetooth link, a network interface, a local or wide area network, and other communications channels.

The computing system 400 may further include Input/Output (I/O) devices 422. Examples may include, but are not limited to a display, keypad, microphone, audio speakers, vibrating motor, LED lights, etc. The I/O devices 422 may receive input from a user and also display an output of the computation performed by the processor 402. In this document, the terms "computer program product" and "computer-readable medium" may be used generally to refer to media such as, for example, the memory 406, the storage devices 408, the removable storage unit 414, or signal(s) on the channel 420. These and other forms of computer-readable media may be involved in providing one or more sequences of one or more instructions to the processor 402 for execution. Such instructions, generally referred to as "computer program code" (which may be grouped in the form of computer programs or other groupings), when executed, enable the computing system 400 to perform features or functions of embodiments of the present invention.

In an embodiment where the elements are implemented using software, the software may be stored in a computer-readable medium and loaded into the computing system 400 using, for example, the removable storage unit 414, the media drive 410 or the communications interface 418. The control logic (in this example, software instructions or computer program code), when executed by the processor 402, causes the processor 402 to perform the functions of the invention as described herein.

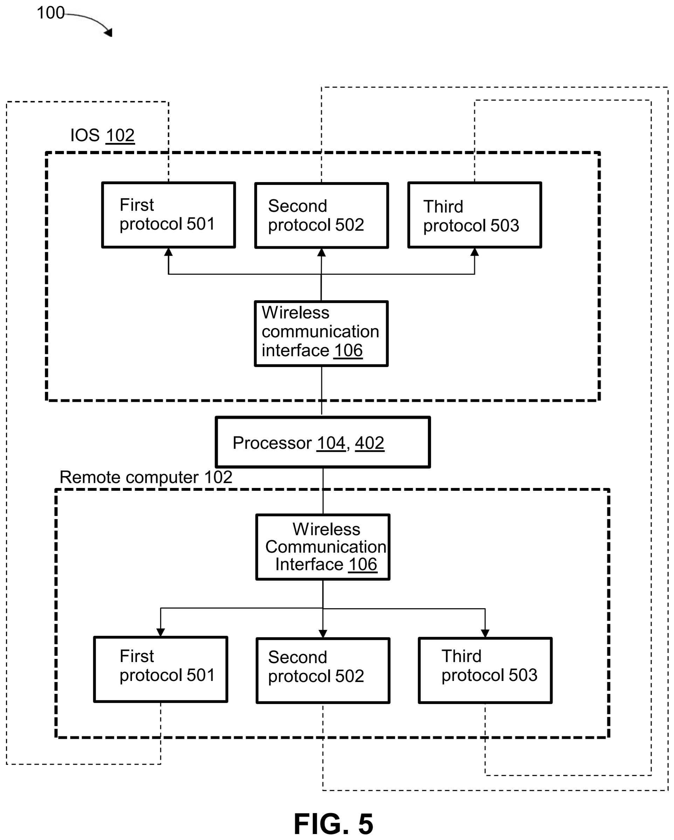

Fig. 5 illustrates an exemplary of the intraoral scanning system 100. In this example, the one or more processors (104, 402) is configured to switch, based on a wireless quality threshold, between the first wireless network protocol 501, the second wireless network protocol 502 and/or a third wireless network protocol 503 to transmit the surface information. Which means that the one or more processors (104, 402) is configured to determine a wireless quality threshold based on the information received by the wireless communication interface 106 of either the intraoral scanning device 102 or the remote computer 102. The intraoral scanning device or the remote computer 102 is the master, and the master decides which of the protocols (501,502,503) that are used for transmissions of information, e.g. surface information, internal information and/or control data. The one or more processors (104, 402) is configured to receive Quality-of-Service data of the wireless communication protocol during wireless transmission in the wireless communication protocol, and the wireless quality threshold includes a level of the Quality-of-Service data. The wireless quality threshold is determined to be a level of Quality-of-Service data which provides an optimal wireless connection to transmit surface information in real time via the second wireless network protocol or the third wireless network protocol.

One or more techniques for generating recommendations for enhancement of an existing legacy or monolith application are disclosed. The techniques provide for a solution for visualizing different data-types, irrespective of each data-type having their respective representation. As such, the techniques allow developers to visualize geometric entities while debugging to thereby aid in code understanding and maintenance. Further, the techniques are not limited by the IDE extendibility or the programming language. Furthermore, the above techniques provide a two-way communication link that allows the debugger to post queries and receive corresponding rendering. The techniques are independent of different IDEs and platforms. The techniques help in reducing overall application (code) development time and effort.

While the invention has been described in connection with a number of embodiments and implementations, the invention is not so limited but covers various obvious modifications and equivalent arrangements, which fall within the purview of the appended claims. Although features of the invention are expressed in certain combinations among the claims, it is contemplated that these features can be arranged in any combination and order.

### Items:

1. An intraoral scanning system (100) comprising:
   an intraoral scanning device (102) configured to acquire first light information and second light information reflected from a dental object (101) during a scanning session,
   one or more processors operably connected to the intraoral scanning device (102), and at least one of the one or more processors is one or more remote computers (104), the one or more processors are configured to:
      determine, in real time, surface information from the first light information,
      generate a three-dimensional (3D) surface model (108) of the dental object (101) using
   the surface information, and
      determine internal information from the second light information of the dental object (101),
   a wireless communication interface (106) configured to communicate between the intraoral scanning device (102) and the one or more remote computers (104) over a wireless communication protocol, wherein the wireless communication protocol includes a first wireless network protocol and a second wireless network protocol,
      wherein the surface information is transmitted via the first wireless network protocol and the internal information is transmitted via the second wireless network protocol.
2. The intraoral scanning system (100) according to item 1, wherein the first wireless network protocol is an unsecured wireless network protocol, and wherein the second wireless network protocol is a secured wireless network protocol.
3. The intraoral scanning system (100) according to any of the previous items, comprising a third wireless network protocol which is a secured wireless network protocol.
4. The intraoral scanning system (100) according to any of the previous items, wherein the one or more processors is configured to generate control data that includes status information of the intraoral scanning device (102), and the control data is transmitted via the second wireless network protocol or the third wireless network protocol.
5. The intraoral scanning system (100) according to any of the previous item, wherein the first wireless network protocol is configured to transmit the surface information in real-time to the one or more remote computers (104), and wherein the 3D surface model (108) of the dental object (101) is rendered in real-time.
6. The intraoral scanning system (100) according to any of the previous items, wherein the one or more processors is configured to switch, based on a wireless quality threshold, between the first wireless network protocol and the second wireless network protocol or a third wireless network protocol to transmit the surface information.
7. The intraoral scanning system (100) according to item 6, wherein the one or more processors is configured to receive Quality-of-Service data of the wireless communication protocol during wireless transmission in the wireless communication protocol, and the wireless quality threshold includes a level of the Quality-of-Service data.
8. The intraoral scanning system (100) according to item 7, wherein the wireless quality threshold is determined to be a level of Quality-of-Service data which provides an optimal wireless connection to transmit surface information in real time via the second wireless network protocol or the third wireless network protocol.
9. The intraoral scanning system (100) according to items 2 or 8, wherein the secured wireless network protocol is a Domain Name System (DNS) protocol or a Multicast Domain Name System (mDNS) protocol.
10. The intraoral scanning system (100) according to item 2, wherein the unsecured wireless network protocol is a User Datagram Protocol (UDP) or a Transmission Control Protocol (TCP).
11. The intraoral scanning system (100) according to any of the previous items, wherein the intraoral scanning device (102) is configured to broadcast, by the wireless communication interface (106), a unique identifier of the intraoral scanning device (102) via the wireless communication protocol, and wherein the one or more remote computers (104) is configured to establish a secured wireless connection to the wireless communication interface (106) based on the broadcasted unique identifier.
12. The intraoral scanning system (100) according to item 11, wherein after the secured wireless connection is established between the intraoral scanning device (102) and the one or more remote computers (104), an IPv4 or IPv6 address is transmitted via a secured wireless network protocol to the one or more remote computers (104).
13. The intraoral scanning system (100) according to any of the previous items, wherein password protected data is transmitted from the intraoral scanning device (102) via the secured wireless connection established between the intraoral scanning device (102) and the one or more remote computers (104).
14. The intraoral scanning system (100) according to any of the previous items, wherein the second light information includes infrared wavelengths and/or ultraviolet wavelengths.
15. The intraoral scanning system (100) according to item 14, wherein during a first time slot of a scanning sequence of the intraoral scanning device (102), the second light information includes infrared wavelengths, and during a second time slot of a scanning sequence of the intraoral scanning device (102), the second light information includes ultraviolet wavelengths.
16. The intraoral scanning system (100) according items 14 or 15, wherein the internal information includes infrared light information, ultraviolet light information and/or visible light information acquired by the intraoral scanning device (102).
17. The intraoral scanning system (100) according to any of items 14 to 16, wherein the internal information includes a composition of infrared light information, ultraviolet information and/or visible light information.
18. The intraoral scanning system (100) according to any of the previous items wherein the first light information is acquired by the intraoral scanning device (102) at a first frame rate, and the second light information is acquired by the intraoral scanning device (102) at a second frame rate, and wherein the second frame rate is lower than the first frame rate.
19. A computer-implementable method, comprising:
   acquiring first light information and second light information during a scanning session,
   determining, in real time, surface information from the first light information,
   determining internal information from the second light information,
   communicating between the intraoral scanning device (102) and one or more processors over a wireless communication protocol, and wherein at least one of the one or more processors is one or more remote computers (104), wherein the wireless communication protocol includes a first wireless network protocol and a second wireless network protocol,
   transmitting, via the first wireless network protocol, the surface information, and transmitting, via the second wireless network protocol, the internal information.

## Claims

1. An intraoral scanning system (100) comprising:
an intraoral scanning device (102) configured to acquire first light information and second light information reflected from a dental object (101) during a scanning session,
one or more processors operably connected to the intraoral scanning device (102), and at least one of the one or more processors is one or more remote computers (104), the one or more processors are configured to:
determine, in real time, surface information from the first light information,
generate a three-dimensional (3D) surface model (108) of the dental object (101) using the surface information, and
determine internal information from the second light information of the dental object (101),
a wireless communication interface (106) configured to communicate between the intraoral scanning device (102) and the one or more remote computers (104) over a wireless communication protocol, wherein the wireless communication protocol includes a first wireless network protocol and a second wireless network protocol,
wherein the surface information is transmitted via the first wireless network protocol and the internal information is transmitted via the second wireless network protocol, and
wherein the wireless connection includes at least a second wireless communication protocol that is different from the first wireless communication protocol, and wherein the first wireless communication protocol includes a first security level and the second wireless communication protocol includes a second security level.

2. The intraoral scanning system (100) according to claim 1, wherein the first wireless network protocol is an unsecured wireless network protocol, and wherein the second wireless network protocol is a secured wireless network protocol.

3. The intraoral scanning system (100) according to any of the previous claims, comprising a third wireless network protocol which is a secured wireless network protocol.

4. The intraoral scanning system (100) according to any of the previous claims, wherein the one or more processors is configured to generate control data that includes status information of the intraoral scanning device (102), and the control data is transmitted via the second wireless network protocol or the third wireless network protocol.

5. The intraoral scanning system (100) according to any of the previous claim, wherein the first wireless network protocol is configured to transmit the surface information in real-time to the one or more remote computers (104), and wherein the 3D surface model (108) of the dental object (101) is rendered in real-time.

6. The intraoral scanning system (100) according to claim 3, wherein the one or more processors is configured to switch, based on a wireless quality threshold, between the first wireless network protocol and the second wireless network protocol or a third wireless network protocol to transmit the surface information.

7. The intraoral scanning system (100) according to claim 6, wherein the one or more processors is configured to receive Quality-of-Service data of the wireless communication protocol during wireless transmission in the wireless communication protocol, and the wireless quality threshold includes a level of the Quality-of-Service data.

8. The intraoral scanning system (100) according to claim 7, wherein the wireless quality threshold is determined to be a level of Quality-of-Service data which provides an optimal wireless connection to transmit surface information in real time via the second wireless network protocol or the third wireless network protocol.

9. The intraoral scanning system (100) according to any of the previous claims, wherein the intraoral scanning device (102) is configured to broadcast, by the wireless communication interface (106), a unique identifier of the intraoral scanning device (102) via the wireless communication protocol, and wherein the one or more remote computers (104) is configured to establish a secured wireless connection to the wireless communication interface (106) based on the broadcasted unique identifier.

10. The intraoral scanning device (100) according to any of the previous claims, wherein the first security level and the second security level are dynamically changing in a level of security depending on a connection quality between the intraoral scanning device and the client device.

11. The intraoral scanning system (100) according to any of the previous claims, wherein password protected data is transmitted from the intraoral scanning device (102) via the secured wireless connection established between the intraoral scanning device (102) and the one or more remote computers (104).

12. The intraoral scanning system (100) according to any of the previous claims, wherein the second light information includes infrared wavelengths and/or ultraviolet wavelengths.

13. The intraoral scanning system (100) according to claim 14, wherein during a first time slot of a scanning sequence of the intraoral scanning device (102), the second light information includes infrared wavelengths, and during a second time slot of a scanning sequence of the intraoral scanning device (102), the second light information includes ultraviolet wavelengths.

14. The intraoral scanning system (100) according to any of the previous claims wherein the first light information is acquired by the intraoral scanning device (102) at a first frame rate, and the second light information is acquired by the intraoral scanning device (102) at a second frame rate, and wherein the second frame rate is lower than the first frame rate.

15. A computer-implementable method, comprising:
acquiring first light information and second light information during a scanning session,
determining, in real time, surface information from the first light information,
determining internal information from the second light information,
communicating between the intraoral scanning device (102) and one or more processors over a wireless communication protocol, and wherein at least one of the one or more processors is one or more remote computers (104), wherein the wireless communication protocol includes a first wireless network protocol and a second wireless network protocol,
transmitting, via the first wireless network protocol, the surface information, and
transmitting, via the second wireless network protocol, the internal information.
